# EUROPÄISCHE PATENTANMELDUNG

(11) **EP 3 878 522 A1**
(43) Veröffentlichungstag der Anmeldung: **15.09.2021**
(21) Anmeldenummer: 20020113.5
(22) Anmeldetag: 09.03.2020
(51) Int. Cl.: A62B 18/02, A62B 18/08, A62B 18/00, A41D 13/11, A61L 9/20

(54) **ATEMSCHUTZMASKE**

(71) Anmelder: Bindhammer, Markus, 86316 Friedberg (DE)
(72) Erfinder: Bindhammer, Markus, 86316 Friedberg (DE)
(74) Vertreter: Patentanwälte Munk

(57) **Zusammenfassung**

Atemschutzmaske mit einem Gesichtsmaskenteil (2) und einer diesem zugeordneten Luft-Desinfektionsvorrichtung (3) mit einem Gehäuse (4), das einen mit der Umgebung kommunizierenden Eingang und einen mit dem Innenbereich des Gesichtsmaskenteils (2) kommunizierenden Ausgang aufweist und dessen Innenraum als mit wenigstens einer UV-Licht emittierenden LED-Lichtquelle (6) versehener Luft-Bestrahlungsraum (16) ausgebildet ist, wird dadurch eine Entkopplung der Anordnung der LED-Lichtquelle (6) vom Aufbau des Gehäuses (4) erreicht, dass die LED-Lichtquelle (6) auf einem Schieber (7) angeordnet ist, der lösbar in einer zugeordneten, gehäuseseitig vorgesehenen, zum Luft-Bestrahlungsraum (16) hin offenen Fassung (8) aufnehmbar ist.

## Beschreibung

Die Erfindung betrifft eine Atemschutzmaske mit einem Gesichtsmaskenteil und einer dem Gesichtsmaskenteil zugeordneten Luft-Desinfektionsvorrichtung mit einem Gehäuse, das einen mit der Umgebung kommunizierenden Eingang und einen mit dem Innenbereich des Gesichtsmaskenteils kommunizierenden Ausgang aufweist und dessen Innenraum als mit wenigsten einer UV-Licht emittierenden LED-Lichtquelle versehener Luft-Bestrahlungsraum ausgebildet ist.

Eine Atemschutzmaske dieser Art ergibt sich aus der WO 2017/014102 A1. Anordnungen dieser Art dienen zur Prophylaxe gegen Schmier- und Tröpfeninfektionen. Bei der bekannten Anordnung ist die LED-Lichtquelle in einen festen Teil des Gehäuses eingebettet, was die Herstellung des Gehäuses und die Montage der LED-Lichtquelle verkompliziert. In Folge der in das Gehäuse integrierten Anordnung der LED-Lichtquelle ist diese von außen nicht zugänglich, was die Wartung und Reinigung erschwert. Ebenso wird hierdurch auch eine von Zeit zu Zeit erforderliche Reinigung des Gehäuses erschwert. Die bekannte Anordnung erweist sich daher als nicht einfach und benutzerfreundlich genug.

Hiervon ausgehend ist es daher die Aufgabe der vorliegenden Erfindung, eine Atemschutzmaske eingangs erwähnter Art so zu verbessern, dass die Nachteile der bekannten Anordnung vermieden sowie eine einfache Herstellung und Montage sowie eine hohe Benutzerfreundlichkeit erreicht werden.

Diese Aufgabe wird erfindungsgemäß dadurch gelöst, dass die LED-Lichtquelle auf einem Schieber angeordnet ist, der lösbar in einer zugeordneten, gehäuseseitig vorgesehenen, zum Luft-Bestrahlungsraum hin offenen Fassung aufnehmbar ist.

Diese Maßnahmen ergeben in vorteilhafter Weise eine bauliche Trennung der Anordnung der LED-Lichtquelle vom Gehäuse und damit eine einfache Herstellbarkeit des Gehäuses sowie eine einfache Montage und gute Zugänglichkeit der LED-Lichtquelle. Durch Betätigung des Schiebers kann die hierauf aufgenommene LED-Lichtquelle einfach in das Gehäuse eingeschoben bzw. aus diesem entnommen werden, was eine einfache Wartung und Reinigung der LED-Lichtquelle sowie des Gehäuses ermöglicht. Dieses kann einfach als Kunststoffteil ausgebildet sein, das zur Reinigung ohne den Schieber in eine Spül- oder Waschmaschine etc. eingelegt werden kann. Dasselbe gilt für den vorzugsweise aus textilem Material bestehenden Gesichtsmaskenteil. Der Schieber samt LED-Lichtquelle kann zur Reinigung etc. extra gehandhabt werden, was einen entsprechend vorsichtigen Umgang mit der LED-Lichtquelle ermöglicht.

Vorteilhafte Ausgestaltungen und zweckmäßige Fortbildungen der übergeordneten Maßnahmen sind in den Unteransprüchen angegeben.

Zweckmäßig kann der Schieber mittels einer zugeordneten Rasteinrichtung lösbar in der Fassung festlegbar sein und ein an ihm angebrachtes, manuell betätigbares Entriegelungsorgan aufweisen. Diese Maßnahmen ermöglichen eine einfache Handhabung des Schiebers und gewährleisten gleichzeitig einen zuverlässigen Halt des Schiebers im Gehäuse und damit eine hohe Betriebssicherheit.

Eine weitere zweckmäßige Maßnahme kann darin bestehen, dass das Gehäuse einen zylinderabschnittförmig ausgebildeten Bereich und einen an dessen Mantel nach Art einer Sekante anschließenden, die Fassung enthaltenden Ansatz aufweist, wobei der Ein- und Ausgang des Luft-Bestrahlungsraums den einander gegenüberliegenden Stirnseiten des Gehäuses zugeordnet sind. Diese Maßnahmen führen zu einer kompakten, sinnfälligen Gestaltung des Gehäuses.

Zweckmäßig kann dabei die Fassung als dem Schieber zugeordnete Führungsflächen enthaltende, einen an die Außenkonfiguration des Schiebers angepasste, lichte Innenkonfiguration aufweisende, mit Luft-Bestrahlungsraum verschnittene Ausnehmung des Ansatzes ausgebildet sein, wobei die LED-Lichtquelle in einem bei eingestecktem Schieber den mit dem Luft-Bestrahlungsraum verschnittenen Bereich der Ausnehmung erreichenden Bereich des Schiebers platziert ist. Diese Maßnahmen ergeben eine einfache Bedienbarkeit des Schiebers.

Zweckmäßig kann das Gehäuse aus zwei jeweils eine Gehäusestirnseite enthaltenden, aneinander festlegbaren Hälften bestehen. Dies ermöglicht eine einfache Herstellung des Gehäuses als Gussformling, vorzugsweise als aus Kunststoff bestehender Spritzgussformling.

Eine weitere, besonders zu bevorzugende Maßnahme kann darin bestehen, dass dem Eingang und dem Ausgang des Gehäuses jeweils voneinander beabstandete, gegenüber der Gehäuseachse geneigte, parallele Lamellen zugeordnet sind. Hierdurch wird sichergestellt, dass die für das menschliche Auge und die Haut gefährliche UV-Strahlung nicht aus dem Gehäuse austreten und das Gesicht des Trägers der erfindungsgemäßen Atemschutzmaske oder die Umgebung treffen kann. Diese Maßnahmen begünstigen daher die Verwendung einer LED-Lichtquelle, die besonders kurzwelliges aggressives UV-Licht aussendet, das eine besonders gute keimtötende Wirkung hat.

Zweckmäßig kann dabei als LED-Lichtquelle eine UVC-Licht mit einer Wellenlänge im Bereich von 250nm bis 260nm, insbesondere von 254nm emittierende UVC-LED mit einer Durchlassspannung von 5V sowie einer Strahlungsleistung von 15mW bei 100mA und einem großen Abstrahlwinkel im Bereich von 120° vorgesehen sein.

Zur Erhöhung der Wirkung der LED-Lichtquelle kann die Innenoberfläche des Licht-Bestrahlungsraums vorzugsweise mittels einer Metallbeschichtung verspiegelt sein. Bei Verwendung eines Kunststoffgehäuses hat die Metallbeschichtung den weiteren Vorteil, dass hierdurch die Kunststoffoberfläche gegen die UV- bzw UVC-Strahlung abeschirmt wird und das Kunststoffmaterial so vor einer durch diese Strahlung verursachten, raschen Alterung bewahrt wird.

Eine weitere vorteilhafte Ausgestaltung der übergeordneten Maßnahmen kann darin bestehen, dass der Schieber im Bereich einer in Einführrichtung hinteren Verdickung mit einer mit jeder auf ihm aufgenommenen LED-Lichtquelle elektrisch leitend verbundenen Buchse versehen ist, die über ein ansteckbares Kabel mit einer tragbaren Energiequelle verbindbar ist. Diese Maßnahmen ermöglichen einen mobilen Einsatz der erfindungsgemäßen Atemschutzmaske und gewährleisten damit eine hohe Benutzerfreundlichkeit.

Zweckmäßig kann die Buchse als USB-Buchse ausgebildet sein, die über ein USB-Kabel mit einer tragbaren Powerbank verbindbar ist. Dieses Maßnahmen ermöglichen eine besonders lange Benutzungszeit.

Weitere vorteilhafte Ausgestaltungen und zweckmäßige Fortbildungen der übergeordneten Maßnahmen ergeben sich aus der nachstehenden Beispielsbeschreibung an Hand der Zeichnung in Verbindung mit den Ansprüchen.

In der nachstehenden beschriebenen Zeichnung zeigen:
- Figur 1: eine perspektivische Ansicht einer erfindungsgemäßen Atemschutzmaske,
- Figur 2: die Anordnung gemäß Figur 1 mit gezogenem Schieber,
- Figur 3: eine Explosionsdarstellung des Gehäuses der Anordnung gemäß Figur 2 mit gezogenem Schieber und
- Figur 4: die Anordnung gemäß Figur 1 im einsatzbereiten Zustand.

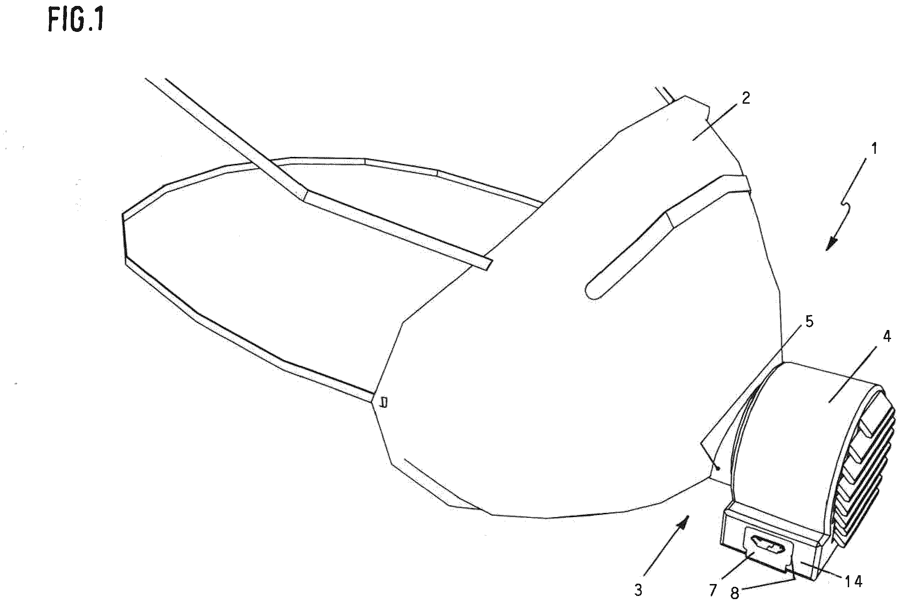

Die der Fig. 1 zugrunde liegende Atemschutzmaske 1 enthält einen Gesichtsmaskenteil 2, welcher vom Träger Mund und Nase bedeckend vor dem Gesicht getragen werden soll, und eine dem Gesichtsmaskenteil 2 zugeordnete Desinfektionsvorrichtung 3 mit einem vorzugsweise lösbar am Gesichtsmaskenteil 2 anbringbaren Gehäuse 4. Der Gesichtsmaskenteil 2 besteht im Wesentlichen aus textilem Material und ist mit einem dem Gehäuse 4 zugeordneten Anschlussstutzen 5 versehen, an welchen das Gehäuse 4 lösbar ansteckbar ist. Der Innenraum des Gehäuses 4 ist als Luft-Bestrahlungsraum zum Desinfizieren bzw. Sterilisieren der hindurchströmenden Luft ausgebildet und besitzt einen mit der Umgebung kommunizierenden Eingang und einen mit dem Innenraum des Gesichtsmaskenteils 2 kommunizierenden Ausgang.

Der Luft-Bestrahlungsraum des Gehäuses 4 ist dementsprechend dem Innenbereich des Gesichtsmaskenteils 2 strömungsmäßig vorgeordnet. Die vom Träger der Atemschutzmaske 1 eingeatmete Luft durchströmt daher zunächst den Luft-Bestrahlungsraum 16 des Gehäuses 4, bevor sie in den Innenbereich des Gesichtsmasktenteils 2 gelangt. Die in den Innenraum des Gesichtsmaskenteils 2 gelangende Luft kann zweckmäßig zur Entfernung von Feststoffen gefiltert werden. Hierzu kann im Bereich des Anschlussstutzens 5 oder/und des Ausgangs oder/und des Eingangs des Gehäuses 4 ein geeigneter Feststofffilter angeordnet sein. In einfachen Fällen ist es aber auch denkbar, auf einen derartigen Filter zu verzichten.

Die den Luft-Bestrahlungsraum 16 des Gehäuses 4 durchströmende Luft wird dort durch Bestrahlung mit keimtötendem UV-Licht behandelt und damit desinfiziert bzw. sterilisiert. Die vom Träger der vorliegenden Atemschutzmaske 1 eingeatmete Luft ist daher steril und, sofern ein Filter vorgesehen ist, auch frei von Feststoffen. Zur UV-Licht-Behandlung der Luft im als Luft-Bestrahlungsraum 16 ausgebildeten Innenraum des Gehäuses 4 findet zweckmäßig kurzwelliges UV-Licht, d. h. sogenanntes UVC-Licht Verwendung, das eine Wellenlänge im Bereich von 100nm bis 300nm, vorzugsweise im Bereich von 250nm bis 260nm, insbesondere von 254nm aufweisen kann. Zur Erzeugung der zur Behandlung der Luft dienenden UV-Strahlung ist dementsprechend wenigsten eine vorzugsweise als UVC-LED ausgebildete LED-Lichtquelle 6 vorgesehen, deren Licht in den Innenraum des Gehäuses 4 abgestrahlt werden kann.

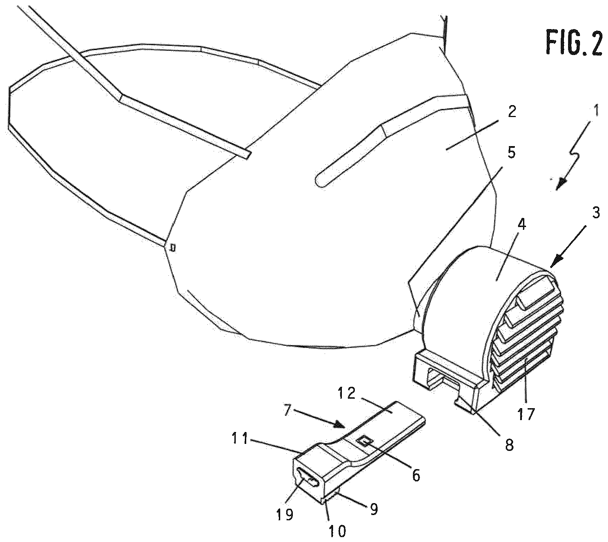

Die Fig. 2 zeigt schematisch eine derartige LED-Lichtquelle 6, die hier auf einem Schieber 7 angeordnet ist, der lösbar in einer zugeordneten Fassung 8 des Gehäuses 4 aufnehmbar ist, welche in ihrem inneren Bereich zum Innenraum des Gehäuses 4 hin offen ist, so dass dieser mit dem UV-Licht der LED-Lichtquelle 6 beaufschlagbar ist. Der Schieber 7 ist in die Fassung 8 einsteckbar und aus dieser heraus abziehbar. In Fig. 1 befindet sich der Schieber 7 im in die zugeordnete Fassung 8 eingesteckten Zustand, in Fig. 2 im herausgezogenen Zustand. Der Schieber 7 ist in seiner eingesteckten Position mittels einer geeigneten Rasteinrichtung lösbar in der Fassung 8 festgelegt. Zum Lösen der Rastverbindung kann der Schieber 7 mit einem in Fig. 2 angedeuteten Entriegelungsorgan 9 versehen sein, das zweckmäßig als manuell betätigbarer Entriegelungsknopf ausgebildet sein kann. Zur Bildung der Rasteinrichtung können im Bereich des Schiebers 7 und der Fassung 8 vorgesehene, in der Raststellung in gegenseitigen Eingriff kommende Rastorgane vorgesehen sein. Im dargestellten Beispiel ist das Entriegelungsorgan 9 hierzu mit Rastzähnen 10 versehen, die in der Raststellung zugeordnete Gegenelemente übergreifen.

Der Schieber 7 besitzt, wie Fig. 2 anschaulich zeigt, eine dem Entriegelungsorgan 9 zugeordnete, in Einfahrrichtung rückwärtige Verdickung 11, von der ein flacher, länglicher, laschenförmiger Ansatz 12 absteht, auf dem die LED-Lichtquelle 6 aufgenommen ist. Die dem Schieber 7 zugeordnete, gehäuseseitige Fassung 8 ist, wie schon erwähnt, zum Innenraum des Gehäuses 4 hin offen. Die auf dem Schieber 7 angeordnete LED-Lichtquelle 6 ist dementsprechend so angeordnet, dass sie sich in der in die Fassung 8 eingesteckten Position des Schiebers 7 in einem zum Innenraum des Gehäuses 4 hin offenen Bereich der Fassung 8 befindet und damit den Innenraum des Gehäuses 4 zur Desinfektion der durchströmenden Luft bestrahlen kann.

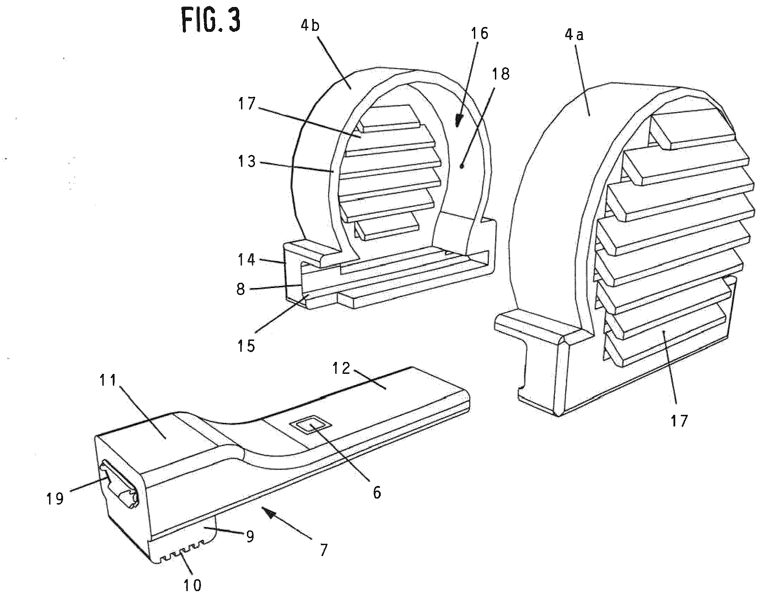

Das Gehäuse 4, das zur Vereinfachung der Herstellung gemäß Fig. 3 aus zwei lösbar aneinander festlegbar Gehäusehälften 4a,4b bestehen kann, besitzt einen zylinderabschnittförmigen Bereich und einen an dessen Mantel 13 nach Art einer Sekante anschließenden Ansatz 14, der die Fassung 8 enthält. Diese ist als mit dem Schieber 7 zugeordneten Führungsflächen 15 versehene, eine an die äußere Konfiguration des Schiebers 7 angepasste, lichte Innenkonfiguration aufweisende, mit dem in Fig. 3 erkennbaren, den Innenraum des Gehäuses 4 gebildeter Luft-Bestrahlungsraum 16 durch verschnittene Ausnehmung des Ansatzes 14 ausgebildet. Die LED-Lichtquelle 6 des Schiebers 7 ist dabei in einem bei in die Fassung 8 eingeführtem Schieber 7 den mit dem Luft-Bestrahlungsraum 16 verschnittenen Bereich der die Fassung 8 bildenden Ausnehmung des Ansatzes 14 erreichenden Bereich des Schiebers 7 platziert. Der Ein- und Ausgang des Gehäuses 4 sind den einander gegenüberliegenden Stirnseiten des Gehäuses 4 zugeordnet. Die beiden Gehäusehälften 4a,4b enthalten dementsprechend jeweils den mit der Umgebung kommunizierenden Eingang oder den mit dem Innenbereich des Gesichtsmaskenteils 2 kommunizierenden Ausgang.

Da die im Luft-Bestrahlungsraum 16 erzeugte UV-Strahlung für das menschliche Auge und die Haut gefährlich sein kann, wird die genannte UV-Strahlung zur Umgebung und zum Innenbereich des Gesichtsmaskenteils 2 hin abgeschirmt. Hierzu sind, wie Fig. 3 weiter zeigt, dem Ein- und Ausgang des Gehäuses 4 jeweils Lamellenanordnungen mit voneinander beabstandeten, gegenüber der Gehäuseachse geneigten, parallelen Lamellen 17 zugeordnet. Die dem hier im Bereich der Gehäusehälfte 4a vorgesehenen Eingang zugeordneten Lamellen 17 können dabei im Bereich der Gehäuseaußenseite vorgesehen sein. Die dem im Bereich der anderen Gehäusehälfte 4b vorgesehenen Ausgang zugeordneten Lamellen 17 sind dagegen zweckmäßig im Bereich der Gehäuseinnenseite vorgesehen, dass sich keine Kollisionen mit dem Anschlussstutzen 5 oder mit im Bereich des Anschlussstutzens 5 des Gesichtsmaskenteils 2 vorhandenen Einbauten, beispielsweise in Form eines Filters etc., ergeben können.

Zur Verstärkung der im Luft-Bestrahlungsraum 16 erzeugten UV-Strahlung kann die Innenoberfläche 18 des Luft-Bestrahlungsraums 16 zweckmäßig verspiegelt sein. Hierzu kann die Innenoberfläche des Gehäuses 4, das als Kunststoffteil hergestellt werden kann, im Bereich seiner Innenoberfläche zweckmäßig mit einer spiegelnden Metallbeschichtung versehen und so gleichzeitig gegen die im Luft-Bestrahlungsraum 16 vorhandene Strahlung abgeschirmt und so gegen Alterung geschützt sein. Es wäre aber auch denkbar, dass das Gehäuse 4 insgesamt aus einem spiegelnden Material besteht.

Das im Luft-Bestrahlungsraum 16 erzeugte UV-Licht ist zweckmäßig kurzwelliges UVC-Licht mit einer Wellenlänge im Bereich von 210nm bis 300nm, vorzugsweise von 250nm bis 260nm, insbesondere von 254nm, wobei letzeres besonders aggressiv und dementsprechend für den vorgesehenen Zweck auch besonders wirksam ist. Die UV-Lichtquelle 6 ist dementsprechend als derartiges UVC-Licht emittierende UVC-LED ausgebildet, wobei eine Durchlassspannung von 5V sowie eine Strahlungsleistung von 15mW bei 100mA vorgesehen sein können. Zweckmäßig ist die LED-Lichtquelle 6 auch so ausgebildet, dass sie einen großen Abstrahlwinkel von etwa 120° aufweist, so dass sich eine gute Ausleuchtung des Luft-Bestrahlungsraums 16 ergibt.

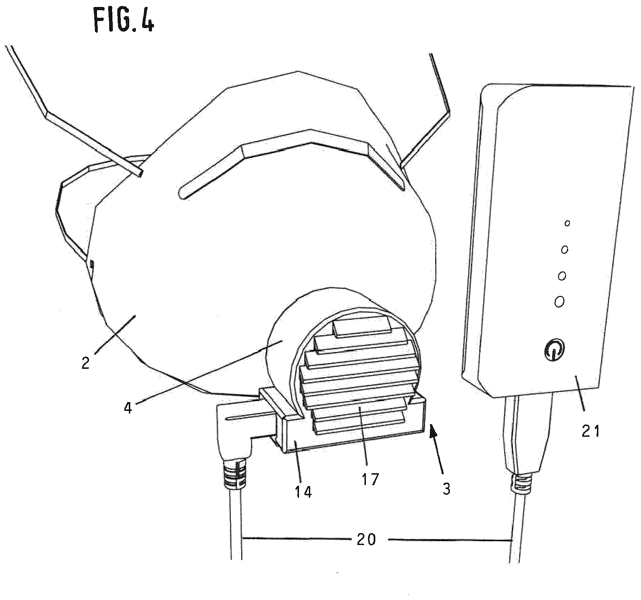

Die LED-Lichtquelle 6 muss mit Strom versorgt werden. Hierzu ist der Schieber 7 gemäß Fig. 2 und 3 im Bereich seiner in Einführrichtung hinteren Verdickung 11 mit einer mit jeder auf dem Schieber 7 aufgenommenen LED-Lichtquelle 6 elektrisch leitend verbundenen Buchse 19 versehen, die gemäß Fig. 4 mittels eines ansteckbaren Kabels 20 mit einer tragbaren, durch eine Batterie gebildeten Energiequelle 21 verbindbar ist. Diese kann zweckmäßig als sogenannte elektronische Powerbank ausgebildet sein, die mittels eines USB-Kabels an die Buchse 19 anschließbar ist. Die Buchse 19 ist dementsprechend zweckmäßig als USB-Buchse und das Kabel 20 als USB-Kabel ausgebildet. Die Ansteuerung der die LED-Lichtquelle 6 bildenden UV-LED bzw. UVC-LED erfolgt zweckmäßig durch Pulsweitenmodulation einer an- und abschwellenden Spannung in Form einer Sägezahnspannung etc.. Alternativ kann auch eine als Konstantstromquelle ausgebildete Schaltung vorgesehen sein. Durch eine derartige indirekte Ansteuerung wird einer Überhitzung der genannten LEDs entgegengewirkt. Bei einer direkten Ansteuerung können diese LEDs sehr heiß werden, so dass eine Kühlung mittels platzaufwändiger Kühlkörper etc. erforderlich ist, was zwecks platzsparender Bauweise verhindert werden soll.

Die UV-Lichtquelle 6 kann zweckmäßig in SMD-Bauweise auf dem Schieber 7 angeordnet sein. Der laschenförmige Ansatz 12 des Schiebers 7 fungiert dementsprechend praktisch als mit der Buchse 19 verdrahtete Leiterplatte, auf welcher die LED-Lichtquelle 6 offen angeordnet sein kann. Die LED-Lichtquelle 6 enthält mindestens eine LED. Es kann aber auch eine Anordnung mit mehreren LEDs vorgesehen sein.

## Patentansprüche

1. Atemschutzmaske mit einem Gesichtsmaskenteil (2) und einer diesem zugeordneten Luft-Desinfektionsvorrichtung (3) mit einem Gehäuse (4), das einen mit der Umgebung kommunizierenden Eingang und einen mit dem Innenbereich des Gesichtsmaskenteils (2) kommunizierenden Ausgang aufweist und dessen Innenraum als mit wenigstens einer UV-Licht emittierenden LED-Lichtquelle (6) versehener Luft-Bestrahlungsraum (16) ausgebildet ist, **dadurch gekennzeichnet, dass** die LED-Lichtquelle (6) auf einem Schieber (7) angeordnet ist, der lösbar in einer zugeordneten, gehäuseseitig vorgesehenen, zum Luft-Bestrahlungsraum (16) hin offenen Fassung (8) aufnehmbar ist.

2. Atemschutzmaske nach Anspruch 1, **dadurch gekennzeichnet, dass** der Schieber (7) mittels einer zugeordneten Rasteinrichtung lösbar in der Fassung (8) festlegbar ist und ein an ihm angebrachter, manuell betätigbares Entriegelungsorgan (9) aufweist.

3. Atemschutzmaske nach Anspruch 2, **dadurch gekennzeichnet, dass** der Schieber (7) eine von einer rückwärtigen Verdichtung (11) abstehende längliche Lasche (12) aufweist, auf der die LED-Lichtquelle (6) in SMD-Bauweise aufgenommen ist.

4. Atemschutzmaske nach einem der vorhergehenden Ansprüche, **dadurch gekennzeichnet, dass** das vorzugsweise aus Kunststoff bestehende Gehäuse (4) einen zylinderabschnittförmig ausgebildeten Bereich und einen an dessen Mantel (13) nach Art einer Sekante anschließenden, die Fassung (8) enthaltenden Ansatz (14) aufweist und dass der Ein- und Ausgang des Luft-Bestrahlungsraums (16) den einander gegenüberliegenden Stirnseiten des Gehäuses (4) zugeordnet sind.

5. Atemschutzmaske nach einem der vorhergehenden Ansprüche, **dadurch gekennzeichnet, dass** die Fassung (8) als dem Schieber (7) zugeordnete Führungsflächen (15) enthaltende, einen an die Konfiguration des Schiebers (7) angepasste, lichte Innenkonfiguration aufweisende, mit dem Luft-Bestrahlungsraum (16) verschnittene Ausnehmung des Ansatzes (14) ausgebildet ist, wobei die LED-Lichtquelle (6) in einem den mit dem Luft-Bestrahlungsraum (16) verschnittenen Bereich der Ausnehmung erreichenden Bereich des Schiebers (7) platziert ist.

6. Atemschutzmaske nach einem der vorhergehenden Ansprüche, **dadurch gekennzeichnet, dass** das Gehäuse (4) zwei jeweils eine Gehäusestirnseite enthaltende, aneinander festlegbare Hälften (4a,4b) aufweist.

7. Atemschutzmaske nach einem der vorhergehenden Ansprüche, **dadurch gekennzeichnet, dass** dem Ein- und Ausgang des Gehäuses (4) jeweils von einander beabstandete, gegenüber der Gehäuseachse geneigte, parallele Lamellen (17) zugeordnet sind.

8. Atemschutzmaske nach Anspruch 7, **dadurch gekennzeichnet, dass** die dem Eingang zugeordneten Lamellen (17) im Bereich der Gehäuseaußenseite und dem Ausgang zugeordneten Lamellen (17) im Bereich der Gehäuseinnenseite vorgesehen sind.

9. Atemschutzmaske nach einem der vorhergehenden Ansprüche, **dadurch gekennzeichnet, dass** jede LED-Lichtquelle (6) als UVC-Licht mit einer Wellenlänge im Bereich von 250nm bis 260nm, insbesondere von 254nm emittierende UVC-LED ausgebildet ist.

10. Atmenschutzmaske nach Anspruch 9, **dadurch gekennzeichnet, dass** jede LED-Lichtquelle (6) eine Durchlassspannung von 5V sowie eine Strahlungsleistung von 15mW bei 100mA und einem großen Abstrahlwinkel im Bereich von 120° aufweist.

11. Atemschutzmaske nach einem der vorhergehenden Ansprüche, **dadurch gekennzeichnet, dass** die Innenoberfläche (18) des Luft-Bestrahlungsraums (16) verspiegelt, bei einem vorzugsweise aus Kunststoff bestehenden Gehäuse (6) mit einer spiegelnden Metallbeschichtung versehen ist.

12. Atemschutzmaske nach einem der vorhergehenden Ansprüche, **dadurch gekennzeichnet, dass** der Schieber (7) im Bereich einer in Einführrichtung hinteren Verdickung (11) mit einer mit jeder hierauf aufgenommenen LED-Lichtquelle (6) elektrisch leitend verbundenen Buchse (19) versehen ist, die über ein ansteckbares Kabel (20) mit einer tragbaren Energiequelle (21) verbindbar ist.

13. Atemschutzmaske nach Anspruch 12, **dadurch gekennzeichnet, dass** die Eergiequelle (21) eine Batterie enthält.

14. Atemschutzmaske nach einem der vorhergehenden Ansprüche 12 oder 13, **dadurch gekennzeichnet, dass** die Buchse (17) als USB-Buchse ausgebildet ist, die über das als USB-Kabel ausgebildete Kabel (20) mit einer tragbaren, die Energiequelle (21) bildenden Powerbank verbindbar ist.

15. Atemschutzmaske nach Anspruch 14, **dadurch gekennzeichnet, dass** die LED-Lichtquelle (6) durch eine als Pulsweitenmodulation oder Konstantstromquelle ausgebildete Schaltung der die Energiequelle (21) bildenden Powerbank ansteuerbar ist.
